# EUROPÄISCHE PATENTSCHRIFT

(11) **EP 0 547 056 B1**
(45) Veröffentlichungstag und Bekanntmachung des Hinweises auf die Patenterteilung: **21.09.1994**
(21) Anmeldenummer: 91910145.1
(22) Anmeldetag: 31.05.1991
(51) Int. Cl.: C12Q 1/48, C12Q 1/70

(54) **VERFAHREN ZUR BESTIMMUNG VON POLYMERASE-AKTIVITÄT**
METHOD FOR THE DETERMINATION OF POLYMERASE ACTIVITY
PROCEDE DE DETERMINATION DE L'ACTIVITE DE POLYMERASES

(30) Priorität: 31.08.1990 DE 4027616
(43) Veröffentlichungstag der Anmeldung: 23.06.1993
(73) Patentinhaber: BOEHRINGER MANNHEIM GMBH, 68298 Mannheim (DE)
(72) Erfinder: EBERLE, Josef, D-8000 München 2 (DE); SEIBL, Rudolf, D-8122 Penzberg (DE); KESSLER, Christoph, D-8021 Dorfen (DE); KÖNIG, Bernhard, D-8137 Berg 3 (DE)
(74) Vertreter: Kolb, Bernd, Dr.
(86) Internationale Anmeldenummer: EP9101016
(87) Internationale Veröffentlichungsnummer: WO9204467

(56) Entgegenhaltungen:
- EP-A- 198 662
- EP-A- 322 922
- EP-A- 324 468
- EP-A- 392 459
- FR-A- 2 611 274
- VIROLOGY, vol. 147, 12 August 1985, Academy Press, New York, NY (US); A.D. HOFFMAN, pp. 326-335#

## Beschreibung

Gegenstand der Erfindung sind ein Verfahren zur Bestimmung von Polymerase-Aktivität, ein Reagenz und ein Reagenzkit zur Ausführung des Verfahrens und die Verwendung des Verfahrens zum Nachweis von HIV, Verfahren zur Ermittlung der Hemmwirkung von Substanzen auf Polymeraseaktivität, Verfahren zum Nachweis von Antikörpern gegen die Polymerase und Verfahren zum Nachweis von Promotorsequenzen.

Polymerasen sind Enzyme, die in Lebewesen eine grundlegende Bedeutung haben. Sie ermöglichen nämlich beispielsweise sowohl die Vermehrung von Nukleinsäuren als auch deren Umsetzung in andere Nukleinsäuren, die für die Synthese von Proteinen benötigt werden. Daher kommen Polymerasen auch in allen Zelltypen vor, beispielsweise auch in Bakterien und auch viele Viren kodieren für eine eigene Polymerase. Ein spezieller Vertreter der Polymerasen ist die Reverse Transkriptase (RT).

Seitdem durch die Entdeckung humanpathogener Retroviren im letzten Jahrzehnt (Science 1983, 220: 868-871, Proc. Natl. Acad. Sci. USA 1980, 77: 7415-7419 und Biochem. Biophys. Res. Comm. 121, 126-133 (1984)) der Nachweis der Reversen Transkriptase (RT) als typischem Leitenzym dieser Virusfamilie immer mehr an Bedeutung gewonnen hat, sind gehäuft Methodenverbesserungen für die Aktivitätsbestimmung der RT beschrieben worden (Virology 1985, 147: 326-335). Zum Einsatz kommen RT-Tests im wesentlichen in drei Situationen:
1. Zur Charakterisierung eines neuisolierten Virus als Retrovirus, und zur Unterscheidung von Retroviren untereinander,
2. zur Bestimmung des Erfolgs einer Virusisolierung aus Untersuchungsmaterial von einem bekanntermaßen Retrovirus-Infizierten oder einem möglicherweise Infizierten und
3. zur Frage der in vitro-Wirksamkeit von Chemotherapeutika gegen diese; für Retroviren essentielle, Enzymfunktion.

Limitierungen für den bekannten RT-Test sind in den ersten beiden Einsatzbereichen die aufwendige Probenvorbereitung (Ultrazentrifugation oder PEG-Fällung, wegen der sonst zu geringen Empfindlichkeit), für den zweiten Einsatzbereich, die Konkurrenz durch sensitive, kostengünstigere und einfache Antigennachweise (ELISA) und alle drei Bereiche betreffend, der Umstand, daß die üblichen RT-Tests mit isotopenmarkierten Nukleotiden arbeiten und damit spezielle Voraussetzungen an das Labor (Ausstattung, Umgangsgenehmigung, Personal, Entsorgung) stellen.

Alle in der Literatur bekannten Verfahren zum Nachweis der Enzymaktivität der Reversen Transkriptase, insbesondere der Reversen Transkriptase aus HIV, beruhen auf Assays, in denen ein Primer, der zu einer Teilsequenz eines Templats komplementär ist, durch den Einbau markierter Nukleotide verlängert wird. Die Abtrennung nicht eingebauter Nukleotidvorstufen von dem verlängerten Primer, der über Wasserstoffbrückenbindungen an das Template gebunden ist, geschieht in der Regel durch Ausfällung des Polymers mit Hilfe von Trichloressigsäure oder Ethanol und anschließender Filtration oder durch Adsorption des Polymers an Membranfilter mit positiv geladener Oberfläche (z.B. an DEAE, wie in Journal of Virological Methods 19, 161-168 (1988) beschrieben) oder durch andere Verfahren, die geeignet sind, ein negativ geladenes Polymer von einem negativ geladenen Monomer abzutrennen. Dies sind sehr aufwendige Maßnahmen, die zusätzliche Handling-Schritte erfordern.

In der WO 90/06373 ist ein Verfahren beschrieben, welches immobilisierte Nukleinsäuren als Template-Nukleinsäuren verwendet. Das Verfahren hat den Nachteil, daß die spezifische Immobilisierung dieser template Nukleeinsäuren ohne Störung der Zugänglichkeit für Enzyme nicht genügend ist. Das Verfahren kann daher durch die verfügbare Menge an reaktionsfähiger Template-Nukleinsäure limitiert werden. Außerdem entstehen bei dem Verfahren eine große Anzahl unterschiedlicher Nukleinsäuren, deren Bindung über die Template-DNS an die Festphase wegen unterschiedlicher Hybridisierungsbedingungen sehr stark variiert. Reaktionen an der Festphase haben außerdem eine schlechtere Kinetik als Reaktionen in Lösung.

Im Falle der quantitativen Bestimmung von HIV-Partikeln hat sich darüber hinaus herausgestellt, daß Immunoassays, mit denen HIV-Antigene nachgewiesen werden, nicht zuverlässig sind (New England Journal of Medicine, Vol. 321, 24, 1673-1675, 1989).

Aus J. Clin. Microbiology 27, 7, 1453-1455 (1989) und AIDS Research and Human Retroviruses 5, 535-540 (1989) ist ein Verfahren zum Nachweis von Antikörpern gegen HIV-RT bekannt, welches auf der Bestimmung von Polymeraseaktivität in Anwesenheit von Antikörpern gegen HIV beruht. Die mit Hilfe der Polymerase gebildeten radioaktiv markierten Nukleinsäuren werden unspezifisch an Membranen immobilisiert. Dieses Verfahren ist außerdem sehr aufwendig.

Aufgabe der Erfindung war es daher, einen schnellen, einfachen, zuverlässigeren und sensitiveren Test auf Polymeraseaktivität zur Verfügung zu stellen. Diese Aufgabe wird durch die Erfindung gelöst.

Gegenstand der Erfindung ist ein Verfahren zur Bestimmung von Polymerase-Aktivität welches folgende Schritte umfaßt:
- Inkubation einer Probe, welche die Polymerase enthält, mit einer Templat-Nukleinsäure und einem nachweisbaren Mononukleosidtriphosphat.
- Abtrennung der gebildeten Nukleinsäuren von dem nachweisbaren Mononukleosidtriphosphat, und
- Nachweis der in den Nukleinsäuren enthaltenen nachweisbaren Nukleotide, wobei
- die Inkubationsmischung zusätzlich ein immobilisierbares Mononukleosidtriphosphat enthält, und
- die Inkubationsmischung mit einer festen Phase in Kontakt gebracht wird, die sowohl das immobilisierbare Mononukleosidtriphosphat als auch die Nukleinsäuren, in welche das immobilisierbare Nukleotid eingebaut ist, selektiv und im wesentlichen vollständig bindet.

Ebenfalls Gegenstand der Erfindung sind Reagenzien zur Durchführung des Verfahrens, Verfahren zur Ermittlung der Hemmwirkung von Substanzen auf Polymeraseaktivität, Verfahren zum Nachweis von Antikörpern gegen die Polymerase und Verfahren zum Nachweis von Promotorsequenzen.

Unter Polymerase-Aktivität wird die Enzymaktivität von Polymerasen verstanden, Mononukleosidtriphosphate unter Zuhilfenahme einer Templat-Nukleinsäure zu einer Nukleotidsequenz zu polymerisieren. Dabei werden die Mononukleotide an eine Nukleinsäuresequenz angehängt und in einen zu einer Nukleinsäuresequenz komplementären Nukleinsäurestrang eingebaut. Je nach Polymerase bildet sich ein Nukleinsäureeinzel- oder -doppelstrang.

Es gibt mehrere Typen von mit dem erfindungsgemäßen Verfahren bestimmbaren Polymerasen, die man nach Art des Substrats bzw. des Polymerisationsprodukts unterschiedlich benennt. Beispiele sind DNS-abhängige RNS-Polymerasen (Typ I), wie T3-, T7- oder E.coli-RNS-Polymerase, DNS-abhängige DNS-Polymerasen (Typ II), wie Klenow-Fragment, RNS-abhängige DNS-Polymerasen (Typ III), wie Reverse Transkriptase, und RNS-abhängige RNS-Polymerasen (Typ IV), die Qβ- und Picornavirus-Polymerase. Analyten im erfindungsgemäßen Verfahren sind beispielsweise die viralen Reversen Transkriptasen, insbesondere HIV-reverse Transkriptase.

Als Templat-Nukleinsäuren werden in der vorliegenden Erfindung Nukleinsäuren bezeichnet, welche ein Substrat der zu bestimmenden Polymerase bzw. des Polymerasetyps darstellen. Substrate für Typ I und Typ II sind daher Desoxyribonukleinsäuren, Substrate für Typ III und IV Ribonukleinsäuren. Substrat für Typ III kann auch Desoxyribonukleinsäure sein, zur Unterscheidung von Typ II wird jedoch bevorzugt Ribonukleinsäure als Substrat verwendet.

Für Typ II und III Polymerasen ist es möglich, als Templat-Nukleinsäuren sowohl heteropolymere als auch homopolymere Nukleinsäuren zu verwenden. Damit die in-vitro Aktivität von Typ II oder Typ III-Polymerasen gemessen werden kann, werden üblicherweise Primer zugesetzt.

Bei Tests auf Typ I-Polymerasen ist die Verwendung einer Nukleinsäure erforderlich, die einen entsprechenden spezifischen Promoter aufweist, beispielsweise den lac-Promoter aus E.coli für E.coli RNA-Polymerase oder Promoter von T7-Phagen für T7-RNS-Polymerase (Chamberlin u Ryan, The Enzyme Vol. XV, S.87-108, 1982). Umgekehrt ist zum Nachweis eines spezifischen Promoters eine spezifische Polymerase erforderlich.

Die Templat-Nukleinsäuren zur Bestimmung von Typ IV-Polymerasen haben ebenfalls spezifische Primer-Strukturen, wie endständig gebundene Proteine, bestimmte Sekundärstruktur etc. Sie hängen von der Polymerase ab und sind dem Fachmann bekannt.

Mononukleosidtriphosphate sind die Triphosphate der Nukleoside mit natürlichen Basen, wie Adenin, Guanin, Cytosin und Uracil bzw. Thymin, oder mit künstlichen Basen, wie Aza- und Deaza-analogen der natürlichen Basen, z.B. 7-Deaza-2'deoxyguanosintriphosphat. Unter Mononukleosidtriphosphaten werden Mononukleotidphosphate oder Monodesoxyribonukleotidtriphosphate verstanden, je nach Typ der zu bestimmenden Polymeraseaktivität. Typ I und IV benötigen Ribonukleotide, Typ II und III Desoxyribonukleotide. Für reverse Transkriptase können sowohl Ribo- als auch Desoxyribonukleotide verwendet werden. Bevorzugt sind hier Desoxyribonukleotide, da die Kombination von Desoxyribonukleotiden mit Ribonukleinsäure als Templat-Nukleinsäure spezifisch für Typ III-Polymerase ist.

Unter nachweisbaren Mononukleosidtriphosphaten im Sinne der Erfindung werden modifizierte Triphosphate verstanden. Die Modifikation ist derart, daß die Mononukleotide neben den natürlichen Mononukleotiden selektiv nachgewiesen werden können, beispielsweise durch photometrische, fluorometrische, radiometrische, enzymatische oder immunologische Reaktionen. Bevorzugt weist das Mononukleosidtriphosphat dazu eine kovalent gebundene nicht radioaktive chemische Gruppe auf. Dies kann ein Farbstoff, ein Fluoreszenzmarker oder eine Komponente einer immunologischen Reaktion, wie ein Antigen, Antikörper oder Hapten sein. Bevorzugt sind Haptene, besonders bevorzugt ist Digoxigenin wegen der hohen erreichbaren Empfindlichkeit. Es wird in diesem Zusammenhang auf die Offenbarung der EP-A-0 324 474 und der EP-A-0 254 090 Bezug genommen.

In der vorliegenden Erfindung wird zusätzlich zu dem nachweisbaren ein immobilisierbares Mononukleosidtriphosphat eingesetzt. Immobilisierbar sind diese Mononukleotide beispielsweise durch kovalent gebundene chemische Gruppen, welche eine spezifische Affinität zu einer festen Phase aufweisen. Bevorzugt ist die chemische Gruppe ein Partner eines spezifischen Bindungspaares. Solche Bindungspaare sind beispielsweise Antigen-Antikörper, Hapten-Antikörper, Antikörper-Antikörper, Vitamin-Bindeprotein, Cofactor-Enyzm und Zucker-Bindeprotein (Lectin). Besonders bevorzugt ist das Paar Vitamin-Bindeprotein, insbesondere Biotin-Avidin oder Biotin-Streptavidin, wobei Biotin die über einen Linker gebundene chemische Gruppe des Mononukleotids darstellt. Das immobilisierbare Mononukleosidtriphosphat sollte, um Ergebnisse im Sinn der Erfindung zu liefern, von dem nachweisbaren Mononukleotid verschieden und unterscheidbar sein. Das Verfahren ist dann erheblich weniger störanfällig und von der Menge an eingebautem Nukleotid unabhängiger als bei Identität dieser Mononukleotide.

Nachweisbares und immobilisierbares Mononukleosidtriphosphat müssen als Substrat von der jeweiligen Polymerase akzeptiert werden, wie für Biotin- und Digoxigenin-Nukleotide gezeigt.

Als Probe des erfindungsgemäßen Verfahrens können alle Flüssigkeiten dienen, die auf ihren Gehalt an Polymerasen untersucht werden sollen. Mit dem Verfahren können Polymerasen in Flüssigkeiten bevorzugt mit einer Konzentration von 10⁻¹² bis zu 10⁻⁸ mol/l, besonders bevorzugt 8x10⁻¹¹ bis 8x10⁻¹⁰ mol/l (ermittelt für RT) bestimmt werden. Solche Lösungen fallen beispielsweise in Verfahren zur Isolierung von Polymerasen, aber auch in Lysaten von Viren und Bakterien oder Zellkulturüberständen an. Die Lysate können sowohl Lysate von Gewebeteilen als auch von Körperflüssigkeiten, wie Blut, sein. Eine weitere Isolierung der Polymerase aus Lysaten kann entfallen. Die Herstellung solcher Proben ist beispielsweise beschrieben in Virology 147, 326, 1985; PNAS, 77, 7415, 1980. Das Verfahren hat durch seine hohe Empfindlichkeit den Vorteil, daß aufwendige Konzentrierungsschritte in den meisten Fällen entfallen können.

In einem ersten Schritt des Verfahrens wird der Probe eine Templatnukleinsäure und mindestens ein nachweisbares Mononukleosidtriphosphat sowie ein immobilisierbares Mononukleosidtriphosphat zugegeben. Wenn eine Polymerase nachgewiesen werden soll, deren Aktivität von der Anwesenheit eines Primers abhängig ist, so wird als Primer ein Oligonukleotid oder Polynukleotid, welches kleiner als die Templat-Nukleinsäure und zu einem Teil der Templat-Nukleinsäure komplementär ist, und welches von der Polymerase um einen zu der Templat-Nukleinsäure komplementären Strangteil verlängert werden kann, eingesetzt. Dabei müssen Bedingungen eingehalten werden, unter denen die Polymerase in der Lage ist, ihre Enzymaktivität zu entfalten. Diese Bedingungen sind für die jeweilige Polymerase verschieden und dem Fachmann bekannt. Dazu gehört beispielsweise die Zugabe einer pH-Puffersubstanz, die den pH-wert in der Nähe des Aktivitätsmaximums der Polymerase festhält, oder auch die Anwesenheit von Kationen, die für die jeweilige Polymeraseaktivität nötig sind. Auch Detergentien, wie Triton X 100, oder Komplexbildner, wie EDTA, können zugegeben werden. Auch die optimale Temperatur ist dem Fachmann bekannt. Zum Nachweis thermophiler Polymerasen, wie Taq-DNS-Polymerase der EP-A-0 258 017, können beispielsweise erhöhte Temperaturen angewandt werden. Für den Fall, daß es sich bei der Templatnukleinsäure um ein Homopolymer handelt, muß das Mononukleosidtriphosphat ein zu dem Nukleotid der Templatnukleinsäure komplementäres Nukleotid enthalten. Bei Polyadenylat als Templatnukleinsäure enthält das Triphosphat bevorzugt einen Thymin- oder Uracilrest, da mit diesen ein zu Polyadenylat komplementärer Nukleinsäurestrang aufgebaut werden kann.

Die Inkubationslösung kann ferner zusätzlich auch das dem nachweisbaren und/oder immobilisierbaren Mononukleosidtriphosphat zugrundeliegende nicht nachweisbare bzw. immobilisierbare Mononukleosidtriphosphat enthalten.

Wenn die Templat-Nukleinsäure ein Copolymer aus allen 4 Mononukleotiden ist, so muß in der Inkubationsmischung jedes der entsprechenden 4 Mononukleosidtriphosphate, sei es in nachweisbarer, immobilisierbarer oder nicht nachweis- oder immobilisierbarer Form, vertreten sein. Bevorzugt ist der Fall, daß die Konzentration der vier Mononukleosidtriphosphate ungefähr gleich ist und ein Teil eines Triphosphats in immobilisierbarer und ein anderer Teil dieses oder eines anderen Triphosphats in nachweisbarer Form vorliegt. Die Gesamt-Konzentration jedes Triphosphats liegt bevorzugt bei 0,0001 bis 10 mmol/l, besonders bevorzugt 0,001 bis 10 mmol/l.

Die Inkubation zum Nachweis von reverser Transkriptase in einer Konzentration von ca. 10⁻¹¹ mol/l beispielsweise kann nach ca. 90 min. bei 35°C beendet werden. Geringere Mengen bis zu 10⁻¹² können durch Verlängerung der Inkubationszeit auf 24 Stunden nachgewiesen werden.

Die Inkubation kann in einem beliebigen Vorinkubationsgefäß, beispielsweise einer Mikrotiterplatte oder einem Tube oder Röhrchen, aber auch schon in Kontakt mit einer festen Phase, an welche das immobilisierbare Mononukleosidtriphosphat oder Nukleinsäuren, die dieses eingehend enthalten, binden können, ausgeführt werden. Bevorzugt ist der Fall, daß das Vorinkubationsgefäß bereits diese feste Phase enthält, also die genannten Verbindungen binden kann. Dann erübrigt sich ein Umfüllen in ein weiteres Gefäß zum Inkubieren mit der festen Phase.

Im Abtrennungsschritt werden die gegebenenfalls durch die Polymeraseaktivität gebildeten Nukleinsäuren, die mindestens ein immobilisierbares und mindestens ein nachweisbares Mononukleotid eingebaut enthalten, an eine feste Phase gebunden und die flüssige Phase entfernt.

Die feste Phase, an welche das immobilisierbare Mononukleosidtriphosphat und Nukleinsäuren, in die es eingebaut wurde, gebunden werden kann, weist den anderen Partner des Bindungspartners auf. Besonders bevorzugt ist die Festphase mit Streptavidin überzogen. Die feste Phase kann in Form eines Behälters oder auch von Kügelchen vorliegen. Typische und bevorzugte Behälter sind Küvetten und Mikrotiterplatten. Zur Verwendung von Kügelchen ist dann für die Immobilisierung ein separater Behälter zur Aufnahme der Inkubationsmischung erforderlich, wenn nicht der Behälter der Vorinkubation verwendet wird.

Die feste Phase muß in der Lage sein, bevorzugt 90% oder mehr der gesamten in der Inkubationslösung enthaltenen Menge an immobilisierbarem Mononukleosidtriphosphat und Nukleinsäure mit eingebautem immobilisierbarem Nukleotid zu binden. Dazu sollte die feste Phase mindestens 90 %, bevorzugt jedoch mehr Bindungsstellen aufweisen, als ursprünglich immobilisierbares Mononukleotid vorhanden war. Da diese Menge bekannt ist, kann der Fachmann leicht die erfoderliche Fläche ermitteln, oder umgekehrt durch Messung der Bindefähigkeit der festen Phase gegenüber Lösungen mit verschiedenen Mononukleotidgehalten die Menge an Mononukleotid bestimmen, die gerade noch von der festen Phase gebunden wird. Es schadet aber der Bestimmung nicht, wenn die feste Phase mehr Bindungsstellen hat, als Mononukleotid eingesetzt wird. Besonders bevorzugte feste Phasen sind Streptavidin-beschichtete Festkörper, wie in der EP-A-0 344578 beschrieben. Durch die Verwendung eines spezifischen Bindungspaares ist sichergestellt, daß wenig unspezifische Bindung von markierten Mononukleosidtriphosphaten vorliegt. Die Bindung von immobilisierbaren Mononukleotiden ist durch die hohe Affinität der oben beschriebenen Bindungspartner nahezu vollständig, insbesondere bei Biotin/Streptavidin.

Im Falle von Biotin/Streptavidin dauert die Inkubation der festen Phase mit dem Inkubationsgemisch bei 35°C vorzugsweise weniger als 2 h. Danach wird die Flüssigkeit von der festen Phase getrennt, beispielsweise durch Abgießen, Abpipettieren oder Absaugen. Dies führt dazu, daß das nicht eingebaute nachweisbare Mononukleosidtriphosphat von dem immobilisierten nachweisbaren Mononukleotid getrennt wird. Zur Vervollständigung der Trennung kann mit einer Waschlösung nachbehandelt werden. Dabei findet wegen der festen Bindung der Nukleinsäuren mit eingebauten immobilisierbaren Nukleotiden an die feste Phase kein wesentliches Auswaschen der immobilisierten Nukleinsäuren statt. Als Waschlösungen kommen beispielsweise einfache Salzlösungen, wie gepufferte und ungepufferte Natriumchloridlösungen, in Frage. Es müssen also nicht Waschlösungen verwendet werden, die die Hybridisierung der Templatnukleinsäuren mit den neugebildeten Nukleinsäuren aufrecht erhalten, wie im Stand der Technik erforderlich.

Anschließend werden die an die feste Phase gebundenen Nukleinsäuren über die ebenfalls eingebauten nachweisbaren Nukleotide bestimmt. Die Bestimmungsmethode richtet sich nach der Art der nachweisbaren Nukleotide. Für den bevorzugten Fall, daß die nachweisbare Gruppe des Mononukleotids eine Komponente einer immunologischen Reaktion ist, wird die feste Phase mit der anderen Komponente der immunologischen Reaktion umgesetzt. Bevorzugt ist diese zweite Komponente markiert, beispielsweise durch ein Enzym oder einen Fluoreszenzmarker. Besonders bevorzugt sind Enzymmarkierungen, beispielsweise mit Peroxidase aus Meerrettich (POD), alkalischer Phosphatase oder β-Galaktosidase. Im bevorzugten Fall, daß die erste Komponente ein Hapten ist, ist die zweite Komponente ein markierter Antikörper gegen dieses Hapten. In diesem Zusammenhang wird auf die WO 89/06698 und die EP-A-0 209 875 bezug genommen. Falls es sich bei dem Marker um ein Direktlabel wie Farbstoff oder Fluoreszenzfarbstoff handelt, kann die Bestimmung des nachweisbaren Mononukleotids direkt visuell oder bevorzugt mit einem Gerät, beispielsweise einem Photometer oder Fluorometer geschehen. Im Falle einer Enzymmarkierung wird die feste Phase mit einem Substrat für dieses Enzym umgesetzt, und diese Reaktion beobachtet, beispielsweise ebenfalls photo- oder fluorometrisch.

Die Menge oder die Anwesenheit von nachweisbarem Mononukleotid an der festen Phase läßt bei dem erfindungsgemäßen Verfahren auf die Menge oder die Anwesenheit der Polymerase oder des Polymerasetyps schließen. Eine quantitative Auswertung kann sich beispielsweise einer Eichkurve bedienen, die mit Proben bekannten Polymerasegehalts aufgenommen wird.

Wenn die Bedingungen, unter denen die einzelnen Polymerasen bzw. Polymerasetypen nachweisbares und immobilisierbares Mononukleotid einbauen, verschieden sind, ist sowohl eine Unterscheidung zwischen einzelnen Polymerasen als auch Polymerasetypen möglich. Es handelt sich daher bei dem erfindungsgemäßen Verfahren um ein selektives Verfahren. Beispielsweise können Polymerasen des einen Typs neben Polymerasen des anderen Typs bestimmt werden.

Das erfindungsgemäße Verfahren hat den Vorteil, daß die markierten Nukleinsäuren in sehr einfacher und vollständiger Weise von den markierten Mononukleosidtriphosphaten abgetrennt werden können. Außerdem kann es als Eintopfreaktion geführt werden. Umfüllschritte können entfallen. Die Möglichkeit der nicht radioaktiven Reaktionsführung erschließt ferner die Vorteile geringen Gerätebedarfs, leichter Automatisierbarkeit und paralleler Bestimmungsdurchführung vieler Proben, beispielsweise in Mikrotiterplatten. Das Verfahren ist äußerst spezifisch, da nur immobilisierbare Nukleotide und Nukleinsäuren mit diesen an die feste Phase gebunden werden. Das Verfahren kann daher mit weniger aufgereinigten Proben auskommen. Eine Bestimmung ist beispielsweise auch in Gegenwart von Bakterien möglich. Eine Trennung des neu gebildeten Stranges von der Templatnukleinsäure ist beispielsweise nicht erforderlich, wenn als nachweisbares Mononukleotid ein Hapten wie Digoxigenin eingesetzt wird. Das erfindungsgemäße Verfahren ist erheblich zuverlässiger, beispielsweise, da die Länge des Produktes der Polymerasereaktion weitaus weniger in das Ergebnis eingeht als bei Verwendung nur eines modifizierten Mononukleotids. Durch die Verwendung unterschiedlich modifizierter Nukleotide kann einerseits die Menge an immobilisierbarem Mononukleotid zugunsten einer quantitativen spezifischen Bindung an die feste Phase klein gehalten und die Menge an nachweisbarem Mononukleotid zugunsten hoher Sensitivität relativ hoch gehalten werden.

In einer bevorzugten Ausführungsform des Verfahrens zur Bestimmung der Reversen Transkriptase wird eine Probe mit Polyadenylat als Templatnukleinsäure, biotinyliertem dUTP (EP-A-0 063 879) als immobilisierbarem und digoxigeniertem dUTP (WO 89/06698) als nachweisbarem Mononukleosidtriphosphat und OligodT als Primer umgesetzt. Nach Inkubation bei ca. 35°C/90 min. wird die Mischung in eine mit Streptavidin beschichtete Küvette umgefüllt. Nach Inkubation von ca. 60 min. wird die Flüssigkeit ausgegossen und die Küvette mit einer Waschlösung gespült. Danach wird eine Lösung von POD-markiertem Antikörper gegen Digoxigenin zugesetzt. Nach einer Zeit von ca. 60 min. wird die Flüssigkeit entfernt und nochmals gespült. Man gibt dann eine Lösung von ABTS zu und mißt die Farbentwicklung bei 405 nm.

Ebenfalls Gegenstand der Erfindung ist ein Reagenz, das in dem erfindungsgemäßen Verfahren eingesetzt werden kann, und eine Templat-Nukleinsäure und mindestens ein nachweisbares und mindestens ein immobilisierbares Mononukleosidtriphosphat enthält. Dieses Reagenz ist bevorzugt polymerasefrei. Bei dem nachweisbaren und dem immobilisierbaren Mononukleosidtriphosphat handelt es sich um unterschiedliche Verbindungen, denen sowohl das gleiche Mononukleotid, beispielsweise Uridin, oder auch verschiedene Mononukleotide, beispielsweise Adenosin (immobilisierbar) und Uridin (nachweisbar) zugrundeliegen konnen. Ist die Templat-Nukleinsäure eine homopolymere Nukleinsäure, beispielsweise Poly-Adenylat, so ist nur eine Art von Mononukleotiden für die nachweisbare und das immobilisierbare Mononukleosidtriphosphat nötig, nämlich das mit einer zu dem Nukleotid der Nukleinsäure komplementären Base (hier beispielsweise Uridin oder Thymidin). Enthält die Templat-Nukleinsäure alle natürlichen Basen, so müssen auch die dazu komplementären Mononukleotide als Triphosphate vorhanden sein. Das Reagenz enthält außerdem bevorzugt Hilfsmittel, wie Wasser als Lösungsmittel, pH-Puffersubstanzen, Detergentien, Stabilisatoren oder Cofaktoren für die Polymerase. Die pH-Puffersubstanzen sollen gewährleisten, daß nach Vereinigung des Reagenzes mit der Probe ein pH-Wert existiert, bei dem die nachzuweisende Polymerase aktiv sein müßte, wenn sie anwesend ist.

Das Reagenz in Form einer wässrigen Lösung enthält die oben angegebenen Bestandteile in folgenden bevorzugten Konzentrationen:

| | |
|---|---|
| Templat-Nukleinsäure | 0.1 bis 2000 »g/ml |
| Mononukleosidtriphosphate (gesamt) | 0,0001 bis 10 mmol/l |
| bevorzugt | 0,001 bis 10 mmol/l |
| nachweisbares Mononukleotid | 0,0001 bis 10 mml, |
| bevorzugt | 0,001 bis 1 mmol/l |
| immobilisierbares Mononukleotid | 0,0001 »mol/l bis 0,1 mmol/l |

Falls ein Primer notwendig ist, ist dieser möglichst in einer Konzentration von 10 ng/ml bis 10 mg/ml, bevorzugt in einer Konzentration von 10-2000 »g/ml vorhanden. Im Falle von Biotin-dUTP als immobilisierbarem und Digoxigenin-dUTP als nachweisbarem Mononukleotid werden diese möglichst in einem Mol-Verhältnis von 1:1 bis 1:50000, im Fall von RT bevorzugt in einem Mol-Verhältnis von 1:5 bis 1:50, besonders bevorzugt von 1:10 bis 1:30 (Bio:Dig) eingesetzt. Diese Werte können für anders modifizierte Nukleotide vom Fachmann entsprechend optimiert werden. Die Werte können auch in Abhängigkeit von der Polymeraseaktivität und der Anzahl der Wandbindungsstellen vom Fachmann entsprechend optimiert werden. Das Reagenz kann jedoch auch in Form einer konzentrierteren Lösung vorliegen, um vor Anwendung verdünnt zu werden. Auch die Aufbewahrung in Form eines Lyophilisates ist möglich. Auch hier wird das Reagenz mit Hilfe einer Lösung rekonstituiert.

Ebenfalls Gegenstand der Erfindung ist ein Reagenzkit zum Nachweis von Polymeraseaktivität, enthaltend in getrennten Behältern:
- Templatnukleinsäure, mindestens ein nachweisbares und mindestens ein immobilisierbares Mononukleosidtriphosphat
- einen Behälter, an welchen das immobilisierbare Mononukleotid selektiv und im wesentlichen vollständig gebunden werden kann; und
- Waschlösungen.

Erforderlichenfalls enthält das Reagenzkit (bevorzugt im Gemisch mit der Templatnukleinsäure) einen Primer. Zusätzlich zum oben geschilderten Reagenz enthält es einen zum Ausführen des Verfahrens erforderlichen Behälter und Waschlösungen. Eine bevorzugte Ausführungsform enthält außerdem für den Nachweis des nachweisbaren Mononukleotids verwendbare Reagenzien. Dazu gehört beispielsweise bei Verwendung eines immunologischen Paares zur Bindung einer Markierung an das nachweisbare Mononukleotid der markierte andere Partner der am nachweisbaren Mononukleotid gebundenen Komponente des immunologischen Paares. Im Falle einer Enzymmarkierung gehören dazu auch ein Enzymsubstrat und gegebenenfalls erforderliche pH-Puffersubstanzen. Das erfindungsgemäße Reagenzkit hat den Vorteil, daß alle Reagenzien stabil und auf relativ geringem Raum unterzubringen sind und ihr Gebrauch einfacher ist als für Reagenzien des Standes der Technik.

Eine bevorzugte Ausführungsform eines Reagenzkits enthalt:
a) Einen Behälter mit Templat-Nukleinsäure, digoxigeninmarkiertem Mononukleosidtriphosphat, biotinmarkiertem Mononukleosidtriphosphat, pH-Puffer, Detergens, Komplexbildner, Cofaktoren, Salze und Oxidationsschutzmittel.
b) Eine mit Streptavidin beschichtete Küvette, ein Röhrchen, Kügelchen oder eine Mikrotiterplatte.
c) Waschlösung
d) Lösung eines POD-markierten Antikörpers gegen Digoxigenin.
e) Ein POD-Substrat, beispielsweise 2,2'-Azino-di-[3-ethyl-benzthiazolinsulfat (6)] (ABTS) in Pufferlösung.

Für einen Nachweis von Typ II- oder III-Polymerasen enthält a) außerdem bevorzugt einen Primer.

Alle Komponenten des Reagenzkits sind stabil, lange haltbar, billig und einfach herzustellen und zu entsorgen.

Das erfindungsgemäße Verfahren zum Nachweis von Polymeraseaktivität kann überall dort verwendet werden, wo die Polymeraseaktivität auf die Zusammensetzung einer Probe Aufschluß gibt. Das ist beispielsweise der Fall zum Nachweis von Polymerasen in Proben, beispielsweise Körperflüssigkeiten oder auch aufgearbeiteten Enzymisolaten, zum Nachweis von polymerasehaltigen Mikroorganismen, insbesondere Bakterien und Viren, zur Typisierung von unbekannten Polymerasen oder Mikroorganismen und zur Überprüfung von Substanzen in ihrer eventuellen Polymerase-Inhibitionswirkung. Die Abnahme der bekannten Polymeraseaktivität bei Zugabe der Substanz ist dabei ein Maß für die Inhibition und eventuelle therapeutische Anwendbarkeit der Substanz als anti-bakterielles oder antivirales Mittel.

Ebenfalls Gegenstand der Erfindung ist ein Verfahren zum Nachweis von HIV, welches auf dem erfindungsgemäßen Verfahren zur Bestimmung von Polymerasen beruht. Dazu wird ein Lysat einer Probe hergestellt, bei der die Anwesenheit von HIV vermutet wird,
- dieses Lysat mit einer Templat-Ribonukleinsäure, mindestens einem nachweisbaren und einem immobilisierbaren Mono-Desoxyribonukleosidtriphosphat und Hilfsstoffen versetzt,
- nach einer Inkubationszeit diese Mischung in einen Behälter überführt, der das immobilisierbare Mononukleotid und eingebautes immobilisierbares Nukleotid selektiv und im wesentlichen vollständig bindet,
- überschüssiges nachweisbares Mononukleosidtriphosphat abgetrennt und
- das gebundene nachweisbare Mononukleotid nachgewiesen.

Bevorzugt findet die erste Inkubation in Anwesenheit eines Primers statt, da es sich bei HIV-RT um eine primerabhängige Typ-III-Polymerase handelt. Die Inkubation der Mischung kann auch schon in dem bindefähigen Behälter stattfinden.

Dieses Verfahren kann analog auch auf den Nachweis anderer Viren, die mit einer Polymerase verbunden sind, übertragen werden. Als Probe für das Verfahren kann ein Virusisolat, z.B. ein Pellet dienen.

Ein weiterer Gegenstand ist ein Verfahren zum Nachweis eines Antikörpers gegen eine Polymerase durch
- Inkubation der Probe, in welcher der Antikörper nachgewiesen werden soll, mit einer Templatnukleinsäure, einem nachweisbaren Mononukleosidtriphosphat und der Polymerase,
- Abtrennung der gebildeten Nukleinsäuren von dem nachweisbaren Mononukleosidtriphosphat und
- Nachweis der in den Nukleinsäuren enthaltenen nachweisbaren Nukleotide, wobei
- die Inkubationsmischung zuätzlich ein immobilisierbares Mononukleosidtriphosphat enthält, und
- die Inkubationsmischung mit einer festen Phase in Kontakt steht oder gebracht wird, die sowohl das immobilisierbare Mononukleosidtriphosphat als auch die Nukleinsäuren, in welche das immobilisierbare Mononukleotid eingebaut ist, selektiv und im wesentlichen vollständig bindet.

Dieses Verfahren basiert damit auf dem Nachweis von Polymeraseaktivität gemäß oben geschilderter Erfindung, wobei jedoch die Reaktionsmischung eine bestimmte Polymeraseaktivität aufweist und diese mit der Polymeraseaktivität nach Zugabe der Probe, von der vermutet wird, daß sie einen Antikörper gegen diese Polymerase enthält, verglichen wird. Wenn ein Antikörper vorhanden ist, der gegen die Polymerase gerichtet ist, wird die Aktivität geringer sein, als wenn der Antikörper nicht enthalten ist. Als Probe kann Blut, Serum, ein Isolat, ein Zellkulturüberstand etc. eingesetzt werden.

Die Menge und Art der Reagentien entspricht daher im wesentlichen denen des Verfahrens zum Nachweis von Polymerase. Die Polymerasekonzentration hängt auch von der Menge des nachzuweisenden Antikörpers ab. Durch wenige Vorversuche kann der Fachmann jedoch eine geeignete Verdünnung der Polymerase finden. Als Anhaltspunkt kann der Nachweisbereich für das erfindungsgemäße Polymerasenachweisverfahren dienen.

Es kann außerdem vorteilhaft sein, vor dem Zusatz der Polymerase die in der den Antikörper enthaltenden Probe ursprünglich enthaltene Polymeraseaktivität zu zerstören. Weitere, jedoch gebräuchliche Reaktionsbedingungen sind dem Fachmann z.B. aus J. Clin. Microbiology Vol. 27, 7, S. 1453-1455 (1989) und AIDS Research and Human Retroviruses Vol. 5, Seite 535-540 (1989) bekannt.

Bei Nachweis auf Antikörper gegen Typ II oder Typ III Polymerasen wird zusätzlich ein entsprechender Primer zugesetzt.

In einer bevorzugten Ausführungsform des Verfahrens zum Nachweis von Antikorpern gegen HIV-RT wird Templatnukleinsaure, biotinyliertes dUTP (EP-A-0 063 879) als immobilisierbares und digoxigeniertes dUTP (WO 89/06698) als nachweisbares Mononukleosidtriphosphat und eine definierte Menge an Polymerasen, z.B. Reverse Transkriptase, entweder aus Viruslysat oder bevorzugt gentechnisch hergestellt, direkt mit zu untersuchendem Serum ca. 60-90 min. bei ca. 37°C inkubiert. Nach Inkubation wird die Mischung in eine mit Streptavidin beschichtete Küvette umgefüllt. Nach Inkubation von ca. 60 min. wird die Flüssigkeit ausgegossen und die Küvette mit einer Waschlösung gespült. Danach wird eine Lösung von POD-markiertem Antikörper gegen Digoxigenin zugesetzt. Nach einer Zeit von ca. 60 min. wird die Flüssigkeit entfernt und nochmals gespült. Man gibt dann eine Lösung von ABTS zu und mißt die Farbentwicklung bei ca. 405 nm.

Die Menge oder die Anwesenheit von nachweisbarem Mononukleotid an der festen Phase läßt bei dem erfindungsgemäßen Verfahren auf die Menge oder die Abwesenheit des Antikörpers gegen die Polymerase schließen. Eine quantitative Auswertung kann sich beispielsweise einer Eichkurve bedienen, die mit Proben bekannten Antikörpergehalts aufgenommen wird.

Wenn die Bedingungen, unter denen die einzelnen Polymerasen bzw. Polymerasetypen nachweisbares und immobilisierbares Mononukleotid einbauen, verschieden sind, ist sowohl eine Unterscheidung zwischen Antikörpern gegen einzelne Polymerasen als auch Polymerasetypen möglich. Es handelt sich daher bei dem erfindungsgemäßen Verfahren um ein selektives Verfahren. Beispielsweise konnen Antikörper gegen Polymerasen des einen Typs neben solchen gegen den anderen Typ bestimmt werden.

Das erfindungsgemäße Verfahren kann beispielsweise zum Nachweis einer Infektion durch einen Mikroorganismus, welcher eine Polymerase enthält oder welcher die Information für die Produktion einer Polymerase enthält, z.B. von Viren, wie HIV, verwendet werden. Das Verfahren weist die Vorteile des Verfahrens zum Nachweis von Polymeraseaktivität auf, es ist einfach, schnell, zuverlässig und sensitiver als die bekannten Verfahren. Zusätzlich hat das Verfahren den Vorteil, daß es ohne aufwendige IgG-Präparation auskommen kann. Serum kann direkt als Probe eingesetzt werden.

Ebenfalls Gegenstand der Erfindung ist ein Verfahren zum Nachweis einer Promotorsequenz, welches folgende Schritte umfaßt:
- Inkubation einer Probe, in welcher die Promotorsequenz nachgewiesen werden soll, mit einem nachweisbaren Mononukleosidtriphosphat und einer Polymerase, welche die Promotorsequenz als Initiator der Polymerasereaktion benötigt,
- Abtrennung der gebildeten Nukleinsäuren von dem nachweisbaren Mononukleosidtriphosphat und
- Nachweis der in den Nukleinsäuren enthaltenen nachweisbaren Nukleotide, wobei
- die Inkubationsmischung zusätzlich ein immobilisierbares Mononukleosidtriphosphat enthält, und
- die Inkubationsmischung mit einer festen Phase in Kontakt steht oder gebracht wird, die sowohl das immobilisierbare Mononukleosidtriphosphat als auch die Nukleinsäuren, in welche das immobilisierbare Mononukleotid eingebaut ist, selektiv und im wesentlichen vollständig bindet.

Auch dieses Verfahren beruht auf der erfindungsgemäßen Bestimmung von Polymeraseaktivität, jedoch wird im Vergleich dazu eine bestimmte Menge an Polymerase zugegeben, jedoch keine Templat-Nukleinsäure zusätzlich zu der in der zu untersuchenden Probe enthaltenen, gegebenenfalls die Promotorsequenz aufweisenden Nukleinsäure zugesetzt. Bei Anwesenheit eines Promotors, der für die zugegebene Polymerase als Initiator wirksam ist, kann die Polymerasereaktion ablaufen, und können markierte Nukleinsäuren nachgewiesen werden. Promotoren, die nachgewiesen werden können, sind beispielsweise der SP6-, der T7- oder der T3-Promotor. Es werden dann die entsprechenden Polymerasen, d.h. SP6-RNA-Polymerase, T7-RNA-Polymerase oder T3-DNA-Polymerase eingesetzt. Der Test ist spezifisch für den jeweiligen Promotor. Solche Tests konnen beispielsweise beim Nachweis von Nukleinsäurekonstrukten in der Molekularbiologie vorteilhaft verwendet werden. Als Probe wird bevorzugt ein DNA-Isolat verwendet.

Die Konzentrationsangaben und Bedingungen für die einzelnen Reagentien richtet sich im wesentlichen nach den im erfindungsgemäßen Polymerasebestimmungsverfahren genannten Werten.

In einer bevorzugten Ausführungsform des Verfahrens wird eine Probe mit biotinyliertem dUTP (EP-A-0 063 879) als immobilisierbarem und digoxigeniertem dUTP (WO 89/06698) als nachweisbarem Mononukleosidtriphosphat sowie einer für den Promotor spezifischen Polymerase umgesetzt. Nach Inkubation bei ca. 35°C/90 min. wird die Mischung in eine mit Streptavidin beschichtete Küvette umgefüllt. Nach Inkubation von ca. 60 min. wird die Flüssigkeit ausgegossen und die Küvette mit einer Waschlösung gespült.

Danach wird eine Lösung von POD-markiertem Antikörper gegen Digoxigenin zugesetzt. Nach einer Zeit von ca. 60 min. wird die Flüssigkeit entfernt und nochmals gespült. Man gibt dann eine Lösung von ABTS zu und mißt die Farbentwicklung bei ca. 405 nm.

Die Menge oder die Anwesenheit von nachweisbarem Mononukleotid an der festen Phase läßt bei dem erfindungsgemäßen Verfahren auf die Stärke oder die Anwesenheit eines Promotors für die Polymerase oder den Polymerasetyp schließen.

Wenn die Bedingungen, unter denen die einzelnen Polymerasen bzw. Polymerase-Subtypen nachweisbares und immobilisierbares Mononukleotid einbauen, verschieden sind, ist eine Unterscheidung zwischen einzelnen Promotern möglich. Es handelt sich daher bei dem erfindungsgemäßen Verfahren um ein selektives Verfahren. Beispielsweise können Promoter des einen Polymerase-Subtyps neben Promotern des anderen Polymerase-Subtyps bestimmt werden. Bei Eukaryonten-Polymerasen vom Typ I gibt es verschiedene Subtypen (RNA-Pol I, II, III), die verschiedene Promoter-Typen erkennen. Weiterhin erkennt ein bestimmter Polymerase-Subtyp verschiedene Promotoren, abhängig von zusätzlichen Faktoren.

Ein weiterer Gegenstand der Erfindung ist ein Reagenzkit zum Nachweis von Antikörpern gegen eine Polymerase, welches eine Templatnukleinsäure, mindestens ein nachweisbares und mindestens ein immobilisierbares Mononukleosidtriphosphat sowie eine Polymerase, gegen die der nachzuweisende Antikörper gerichtet ist, enthält. Weitere Bestandteile sind bevorzugt Hilfsstoffe und gewünschtenfalls ein Gefäß oder eine sonstige Festphase, die immobilisierbares Mononukleosidtriphosphat und dieses enthaltende Nukleinsäuren im wesentlichen vollständig und selektiv binden. Bevorzugt ist mindestens die Polymerase in einem getrennten Behälter aufbewahrt.

Fig. 1 zeigt die prozentuale RT-Inhibition (Abszisse) durch anti-HIV-positive und anti-HIV-negative Seren (1:40) mit zwei verschiedenen Viruslysatkonzentrationen.

Fig. 2 zeigt die prozentuale RT-Inhibition (Abszisse) durch anti-HIV-positive und anti-HIV-negative Seren (1:40) mit rekombinanter RT (ca. 1:27 000).

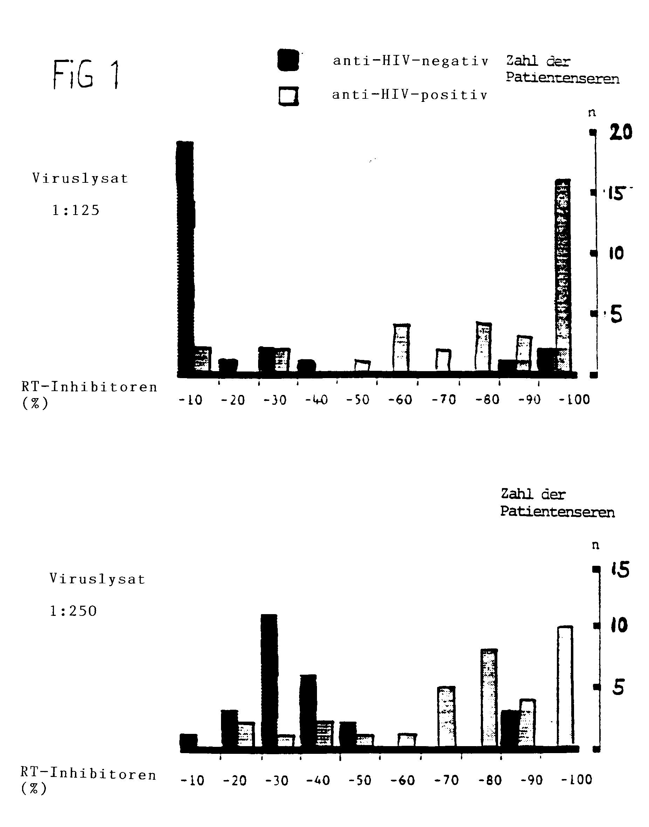

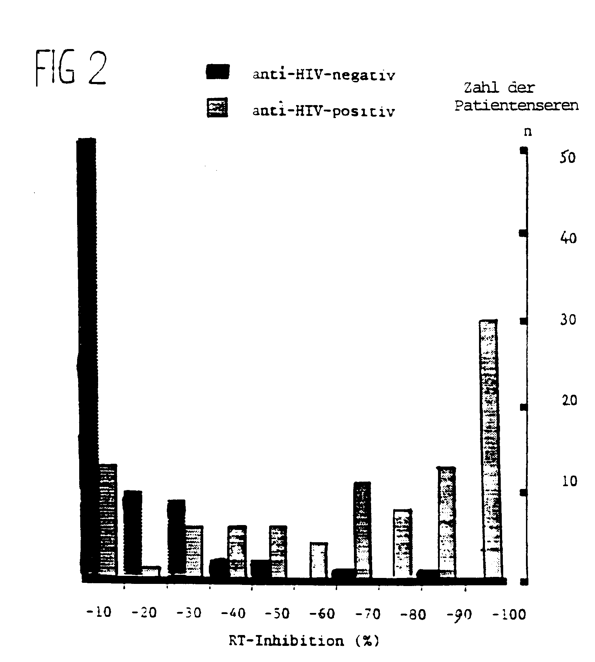

n in Fig. 1 und 2 ist die Anzahl der Seren.

Folgende Beispiele erläutern die Erfindung näher:

### Beispiel 1

### Nachweis von HIV

a) Herstellung von pelletiertem HIV-Virus.
2-3 ml Zellkulturüberstand aus HIV-Viruskulturen oder Virusisolierungsansätzen wird zellfrei gewonnen. Eine anschließende Ultrazentrifugation bei 20.000 g für 2 Stunden führt zur Bildung eines Pellets aus Zellorganellen und (im positiven Fall) Viruspartikeln.
b) Lyse des pelletierten Virus
Das pelletierte Virus wird in 40 »l Lysispuffer gelöst. Lysispuffer:
1,4-Dithioerythrit (DTT, Merck, Darmstadt) 2,5 mM;
EDTA (Merck, Darmstadt) 0,75 mM;
KCl (Merck, Darmstadt) 80 mM;
TRIS HCl (Boehringer Mannheim) 50 mM; pH 8,0 (22°C)
Triton^{R} X-100 (Sigma, Saint Louis) 0,5%
Alternativ können auch 35 »l virushaltiger Überstand mit 5 »l achtfach konzentriertem Lysispuffer versetzt werden.
Dieses Viruslysat ist ohne nennenswerten Aktivitätsverlust bei 2°C etwa 1 Woche lagerfähig (Austrocknen muß verhindert werden).
c) Inkubations/Polymerasereaktion
Zu oben genanntem Lysat werden 20 »l einer Lösung folgender Zusammensetzung zugegeben:
DTT 10 mM;
KCl 290 mM;
MgCl₂ 30 mM;
dTTP 8,3 »M;
Biotin-16-dUTP (Boehringer Mannheim, Best.Nr. 1093070) 125 nM;
DIG-11-dUTP (Boehringer Mannheim, Best. Nr. 1093088) 2,5 »M;
Polyadenylsäure. Pentadekathymidylsäure (Poly A).
(dT)₁₅, (Boehringer Mannheim, Best. Nr. 108677) 700 U/l.
[35 »g/ml]
Tris 50 mmol/l. pH 8,0 (22°C)
Die Mischung wird für 90 min. bis 24 Stunden und länger, je nach gewünschter Testsensitivität bei 35°C inkubiert.
d) Isolierung der Reaktionsprodukte
Nach der Inkubation wird das Reaktionsgemisch in Vertiefungen von Streptavidin-beschichteten 96-well-ELISA-Platten (hergestellt nach EP-A-0 344 578, Kapazität ca. 20 ng Biotin/ml, d.h. in 60 »l ca. 1,2 ng Biotin) überführt und bei 35°C für 60 min. inkubiert.
e) Waschen
Danach werden die Vertiefungen 5mal mit einer Waschlösung (Natriumchlorid 0,85 mol) gewaschen. Die restliche in den Vertiefungen verbliebene Flüssigkeit wird durch Ausklopfen der Platte entfernt.
f) Inkubation mit POD-markiertem Antidigoxigenin-Antikörper
Man pipettiert in jede Vertiefung 200 »l einer Konjugatlösung folgender Zusammensetzung:
Antidigoxigenin-POD (Fabfragmente, Bestellnr. 1207733 Boehringer Mannheim) 200 U/l;
Tween 20 0.5 %
Natriumchlorid 0,85 M
in 0,1 mol/l Natriumphosphatpuffer pH 7,5.
Nach 60 Minuten wird die Konjugatlösung abgegossen und die Vertiefungen 5 mal mit einer Waschlösung
(NaCL 0,85 M) gewaschen.
g) Farbreaktion
In jede Vertiefung werden 200 »l Substratlösung (ABTS (2'2-Azino-di-[3-ethylbenzthiazolinsulfonat (6))] 1g/l in ABTS Puffer (Best. Nr. 1112597, Boehringer Mannheim) pipettiert. Man mißt nach weiteren 60 min.
Reaktionszeit bei 35°C die Farbentwicklung im Photometer bei 405 nm gegen 450 nm.
h) Empfindlichkeit des Tests
Die Empfindlichkeit des erfindungsgemäßen Verfahrens wurde an seriellen Verdünnungen von Aliquots einer positiven Kontrolle (Lysat von pelletierten HIV-I-Partikeln aus ca. 2 ml Überstand einer gut produzierenden Dauerkultur permanenter T-Zellen) untersucht.

**Tabelle 1**

| Virusverdünnung | OD 405nm | Virusverdünnung | OD 405nm |
|---|---|---|---|
| 1:5 | > 2,500 | 1:25 | 1,900 |
| | > 2,500 | | > 2,500 |
| | > 2,500 | | > 2,500 |
| | > 2,500 | | > 2,500 |
| | > 2,500 | | 1,847 |
| | > 2,500 | | 1,691 |
| | > 2,500 | | > 2,500 |
| | > 2,500 | | 1,935 |
| 1:50 | 0,730 | 1:125 | 0,602 |
| | 0,868 | | 0,633 |
| | 0,793 | | 0,599 |
| | 0,880 | | 0,613 |
| | 0,812 | | 0,506 |
| | 0,838 | | 0,496 |
| | 0,769 | | 0,453 |
| | 0,990 | | 0,455 |
| 1:250 | 0,350 | 1:500 | 0,243 |
| | 0,348 | | 0,228 |
| | 0,340 | | 0,274 |
| | 0,404 | | 0,252 |
| | 0,307 | | 0,247 |
| | 0,308 | | 0,230 |
| | 0,242 | | 0,163 |
| | 0,245 | | 0,147 |

| Negativkontrolle | OD 405nm | Leerwert | OD 405nm |
|---|---|---|---|
| 1:2 | 0,130 | | 0,072 |
| | 0,160 | | 0,075 |
| | 0,138 | | 0,068 |
| | 0,119 | | 0,090 |
| | 0,133 | | 0,083 |
| | 0,187 | | 0,073 |
| | 0,121 | | 0,065 |
| | 0,183 | | 0,071 |
| OD = Optische Dichte, gemessen bei 405nm gegen 450nm. Leerwert= Optische Dichte des nicht umgesetzten Substrats. Die angegebenen Meßwerte stammen aus mindestens vier, an verschiedenen Tagen durchgeführten Experimenten. Die benutzten Reaktionsgemische wurden zwischen den Tests bei -20° C gelagert. Es wurden mindestens zwei an verschiedenen Tagen hergestellte Chargen benutzt. | | | |

Dabei zeigt sich regelmäßig bis zu einer 1:5 Verdünnung, bei einigen Testläufen sogar bis zur 1:25 Verdünnung ein maximales Signal (OD > 2,5). Bei kontinuierlichem Abfall des Signals mit steigender Verdünnung liegt der Grenzwert für den Nachweis der reversen Transkriptase etwa bei einer Verdünnung von 1:500 bei 90 min. Reaktionszeit. Bei 24 Stunden Reaktionszeit liegt der Grenzwert bei einer Verdünnung von 1:3000 bis 1:7500 (6 bis 15 mal sensitiver). Die Schwankungen zwischen den 0D-Werten in verschiedenen Testläufen liegen typischerweise bei einem Faktor von kleiner als 2. Verglichen mit der herkömmlichen Methode ist der neue Test bei 90 min. Reaktionszeit mindestens gleich empfindlich wie das herkömmliche Verfahren und zeichnet sich durch eine einfachere Handhabung aus. Die hohe Spezifität des erfindungsgemäßen Verfahrens erlaubt es, im Unterschied zur herkömmlichen Methode, die Reaktionszeit auf mindestens 24 Stunden auszudehnen und damit eine wesentlich höhere Sensitivität zu erhalten. Selbst massiv mit Hämoglobin aus lysierten Erythrozyten bzw. bakteriell kontaminierte Proben zeigen dabei keine über den Grenzwert erhöhten Leerwerte. Beim direkten Paralellansatz von Virusisolierungsansätzen (Pellet aus ca. 3 ml Überstand) konnte das neue Verfahren durch hohe Zuverlässigkeit überzeugen.

### Beispiel 2

### Verfahren zum Nachweis reverser Transkriptase und Überprüfung von Proben in ihrer eventuellen Polymerase-Inhibitionswirkung

Dieser Test verwendet, wie Beispiel 1, einen Test auf reverse Transkriptase.
a) Probenvorbereitung
   Die auf die Anwesenheit von Inhibitoren zu untersuchenden Proben werden üblicherweise in Konzentrationen von 1 bis 10 mg/ml in reinem DMSO (Dimethylsulfoxid) gelöst. Von diesen Stammlösungen werden durch Verdünnung mit RT-Puffer (50 mM Tris-HCl, pH 7,9; 50 mM KCl; 5 mM MgCL₂; 1 mM DTT) verdünnte Lösungen hergestellt (in der Regel 20- bis 200-fache Verdünnung).
b) Inkubation
   Der Test wird direkt in den Streptavidin-beschichteten 96-well-ELISA-Platten (hergestellt nach EP-A-0 344 578) durchgeführt.
   Die folgenden Lösungen werden nacheinander in die Vertiefungen der Platte pipettiert:
   20 »l verdünnte Probenlösung, bzw. RT-Puffer für Positiv-Kontrolle
   20 »l RT-Puffer
   20 »l Template/Primer Komplex, bestehend aus 1 »g RNS-Template (HIV-LTR-5'-gag Fragment) und 21.3 ng
   18-mer DNS-Primer der Sequenz: SEQ ID NO 1 (5'-GTCCCTGTTCGGGCGCCA-3'), 10-fach mit RT-Puffer verdünnt, nachdem zuvor beide Komponenten als Mix in 50-fach konzentrierter Form bei 66.5°C im Wasserbad inkubiert und langsam auf ca. 30°C abgekühlt wurden.
   20 »l HIV-1 RT, nach bekannten Verfahren kloniert und in E.coli experimentiert, zur Homogenität gereinigt (Heterodimer, p66/p51, Science 231, 1289-1291/1986) und in einer Konzentration von 1 »g/ml in RT- Puffer gelöst.
   20 »l Reaktions-Startlösung, bestehend aus
   RT-Puffer (s.o.)
   5 »M dATP
   5 »M dCTP
   5 »M dGTP
   5 »M Dig-11-dUTP
   0,25 »M Biotin-16-dUTP
   0.1 % Nonidet^{R} P 40 (Pierce, Best. Nr. 28324)
   Das 1078 Basen lange, als Template dienende HIV-LTR-5'-gag RNS-Fragment wurde nach bekannten Verfahren durch in vitro-Transkription mit Hilfe der T7-RNS-Polymerase hergestellt.
   Die Mischung wird eine Stunde bei 37°C im Wasserbad einer gemäß EP-A-0 344 578 hergestellten Mikrotiterplatte (Biotin-Bindekapazität ca. 20 ng/ml, d.h. in 100 »l ca. 2 ng Biotin) mit flachem Boden inkubiert. Die Mikrotiterplatte ist dabei mit einer Klebefolie bedeckt.
c) Die Vertiefung wird 5mal mit je 0.3 ml einer Waschlösung (PBS ohne Ca und Mg (Bestellnr. 210 340 Boehringer Mannheim) Tween 20 (Bestellnr. 170-6531, Bio-Rad) 0.05 % (v/v) gewaschen.
d) In jede Vertiefung werden 100 ul Antidigoxigenin-POD (Fabfragmente, Bestellnr. 1207733 Boehringer Mannheim) in einer Lösung (100 mM Tris-HCl, pH 7.9; 150 mM NaCl) zugegeben. Es wird 30 min. bei Raumtemperatur inkubiert.
e) Die Vertiefungen werden 5mal mit je 0.3 ml Waschlosung gewaschen.
f) In jede Vertiefung werden 100 ul folgender Losung zugegeben:
   ABTS 1 g/l in
   ABTS-Puffer (Bestellnr. 111 4597 Boehringer Mannheim) Es wird 20 bis 30 min. bei Raumtemperatur unter Schütteln inkubiert und anschließend die Farbentwicklung bei 405 nm in einem ELISA-Photometer (MR 5000, Dynatech, Referenzfilter 630 nm) gemessen. Die Anwesenheit eines Inhibitors der Polymerase-Reaktion ist anhand der abgeschwächten oder fehlenden Farbentwicklung bei 405 nm zu erkennen.

### Beispiel 3

### Nachweis von spezifischer DNA-abhängiger RNA-Polymeraseaktivität und spezifischen Promotorsequenzen für RNA-Polymerasen auf DNA.

Drei DNA-Fragmente, die entweder den Promoter für die SP6-RNA-Polymerase, die T7-RNA-Polymerase oder die T3-RNA-Polymerase tragen, werden in Transkriptionsansätzen mit einer Ribonukleotidmischung, die auch Dig UTP und Bio UTP enthält, sowie entweder SP6-RNA-Polymerase oder T7-RNA-Polymerase,
inkubiert. In den Ansätzen, in denen Promotor und Polymerase spezifisch zueinander passen, werden die Nukleotide komplementär zur DNA in eine RNA-Kette eingebaut. Nach der Inkubation werden die Reaktionsansatze in Vertiefungen von Streptavidin-beschichteten 96 well-ELISA-Platten überführt. Eingebaute und nicht eingebaute Biotin-Reste binden an Streptavidin. Eine RNA-Polymeraseaktivität kann nachgewiesen werden, indem die dann kovalent mit Biotin-Nukleotiden verknüpften Digoxigenin-Nukleotide bzw. das Digoxigenin-Hapten durch Anti-Digoxigenin-Antikörper nachgewiesen werden. Analog kann ein spezifischer Promotor für eine Polymerase nachgewiesen werden.
a) Transkriptionsansatz:

| | | |
|---|---|---|
| 1 »l | Template-DNA (100 ng/»l), | 100 ng |
| 2 »l | Dig-Bio-Nukleotid-Mix 10 x | |
| 0,5 »l | RNAsin (50 U/»l, Best. Nr. 799017, Boehringer Mannheim), | 25 U |
| 2 »l | Transkriptionspuffer 10 x | |
| x »l | Phagen-RNA-Polymerase | (20 U) |
| ad 20 »l | H₂O | |
| 20 »l | | |

Template-DNAs:
Plasmide, die die entsprechenden Promotor-Sequenzen tragen, wurden durch Restriktionsenzyme geschnitten und auf einem Agarose-Gel elektrophoretisch aufgetrennt. DNA-Fragmente, die jeweils einen Promotor enthielten, wurden aus dem Gel eluiert und als Template-DNAs eingesetzt.
a) Template mit SP6-Promotor:
   Plasmid pSPT18 (Best. Nr. 909 793, Boehringer Mannheim) verdaut mit Eco R I + Ssp I, 951 bp-Fragment isoliert, Transkriptlänge: 58 b
b) Template mit T7-Promotor:
   Plasmid pSPT18 (Best. Nr. 909 793, Boehringer Mannheim) verdaut mit Hind III + Ssp I, 2204 bp-Fragment isoliert, Transkriptlänge: 61 b
c) Template mit T3-Promotor (Kontrolle):
   Plasmid pT3T7lac (Best. Nr. 1014692, Boehringer Mannheim) verdaut mit Eco R I + Sca I, 1815 bp-Fragment isoliert, Transkriptlänge: 60 b
Dig-Bio-Nukleotid-Mix 10 x:

| | | |
|---|---|---|
| 5 »l | 100 mM ATP | 10 mM |
| 5 »l | 100 mM GTP | 10 mM |
| 5 »l | 100 mM CTP | 10 mM |
| 3,25 »l | 100 mM UTP | 6,5 mM |
| 8,75 »l | 1 mM Bio-11-UTP | 175 nM |
| 16,5 »l | 10 mM Dig-11-UTP | 3,3 mM |
| 6,5 »l | H₂O | |
| 50 »l | | |

Transkriptionspuffer 10 x:

| | | |
|---|---|---|
| 400 »l | 1 M Tris/HCl pH 7,9 | 400 mM |
| 60 »l | 1 M MgCl₂ | 60 mM |
| 100 »l | 1 M DTT | 100 mM |
| 20 »l | 1 M Spermidin | 20 mM |
| 420 »l | H₂O | |
| 1 ml | | |

Phagen-RNA-Polymerasen:
- SP6-RNA-Polymerase, Best. Nr. 810 266, Boehringer Mannheim
- T7-RNA-Polymerase, Best. Nr. 881 767, Boehringer Mannheim
Der 10 x-Transkriptionspuffer wurde erst unmittelbar vor Enzymzugabe zugegeben, da DNA mit Spermidinkonzentrationen ab 4 mM gefällt werden kann [Krieg, P. A., Melton, D. A. (1987) Meth. Enzym. 155]. H₂O wurde mit DEPC (Diethylpyrocarbonat) behandelt, um RNAsen zu inaktivieren.
Inkubation 2 h bei 30 °C
[nach Krieg, P. A.; Nucl. Acids Res. 18, 6463 (1990)].
b) Anlagerung der Transkripte an Streptavidin in Mikrotiterplatten
Die Transkriptionsansätze wurden mit 80 »l TE (10 mmol/l TrisHCl, 1 mmol EDTA, pH 8,0) auf 100 »l aufgefüllt.
Jeweils 20 »l des verdunnten Transkriptionsansatzes werden in eine Vertiefung einer Streptavidin-beschichteten 96-well-ELISA-Platte mit flachen Vertiefungen (hergestellt nach EP-A-O 344 578) pipettiert und 180 »l einer 0,05 % (v/v) Tween 20-Lösung zugegeben. Die Platten sind während aller folgenden Anlagerungs- und Reaktionsschritte mit Deckeln (Nunc, Best. Nr. 263339) abgedeckt.
Die Anlagerung erfolgt bei 37 °C unter Schütteln für 1 h.
Ungebundene Reaktionskomponenten werden durch 6maliges Waschen mit jeweils 300 »l einer 0,9%igen NaCl-Lösung entfernt.
c) Nachweis mit Anti-Digoxigenin-Antikörperkonjugat und Farbreaktion
Wie in Beispiel 1 beschrieben, werden 200 »l Anti-Digoxigenin-POD (200 U/l, Best. Nr. 1207733 Boehringer Mannheim) zugegeben. Nach 60 Minuten wird 6mal mit 0,9 % NaCl gewaschen. Ebenfalls wie in Beispiel 1 beschrieben werden 200 »l Substratlösung (ABTS) für 5 bis 60 Minuten in den Vertiefungen bei 37 °C inkubiert und die Farbentwicklung bei 405 nm im Photometer gemessen.

### Beispiel 4

### Nachweis von Antikörpern gegen HIV-RT in Patientenseren mit inhibierender Wirkung auf die RT-Funktion

Dieser Nachweis dient als prognostischer Faktor für eine HIV-Infektion und kann zur Unterscheidung verschiedener Varianten von HIV (HIV 1 und HIV 2) herangezogen werden.

Zu 30 »L Polymeraseverdünnung (entweder aus Viruslysat, oder aus gentechnisch hergestellter reverser Transkriptase) in 1xkonzentriertem Lysispuffer (siehe Beispiel 1) werden 10 »L Serumverdünnung (1:10 in Lysispuffer) zugegeben und für 60-90 Minuten bei 35° C inkubiert. Anschließend gibt man 20 »L der Reaktionslösung (wie in Beispiel 1 unter C beschrieben) zu und inkubiert bei 35° C für 24 Stunden. Im weiteren folgt man dem in Beispiel 1 beschriebenen Verfahren. Falls in der Probe Antikörper, die die reverse Transkriptase funktionell inhibieren, vorhanden sind, wird das Signal unterdrückt oder teilweise reduziert sein. Bei Serumproben ohne RT-inhibierende Antikörper erscheint ein Signal, das der eingesetzten RT-Menge entspricht.
a) Vorversuche:
   Zum Einstellen der optimalen Serum- und Viruskonzentrationen wurden mehrere Versuche durchgeführt. Dazu wurden je sechs HIV-1-positive und HIV-negative Seren in Verdünnung 1:32 und 1:64 mit einer 1:250er Verdünnung eines Standardviruslysats (lysiertes HIV-1 aus ca. 1 mL Überstand einer gut produzierenden Zellkultur von HIV-1 infizierten H9-Zellen) getestet. In einem anderen Versuch wurden je drei weitere HIV-1-positive und HIV-negative Seren 1:32, 1:64 und 1:128 verdünnt mit Standardviruslysatverdünnungen von 1:125, 1:250 und 1:500 getestet. Zusätzlich wurden verschiedene Verdünnungen einer Präparation von gentechnisch hergestellter (rekombinanter) RT (Fa. Mikrogen, München) getestet. Zur Einstellung der optimalen Polymerase-Verdünnung wurden Seren benutzt, die bezüglich ihrer RT-inhibierenden Kapazität vorher charakterisiert waren. Verdünnungen von ca. 1:25000 der rekombinanten RT waren am besten geeignet.
   Die dabei erhaltenen Werte zeigten bei allen HIV-1-positiven Seren eine deutliche Inhibierung der RT-Funktion, während die HIV-negativen Seren keine Beeinträchtigung der RT aufwiesen. Die relative Signalunterdrückung war bei den Serumverdünnungen 1:32 und 1:64 am ausgeprägtesten. Die ideale Verdünnung unseres Standardviruslysats lag zwischen 1:125 und 1:250.
b) Validierung des Testprinzips mit Patientenseren.
   Basierend auf den Erfahrungen aus den Vorversuchen wurden in zwei Ansätzen 34 HIV-1 positive und 26 HIV-negative Seren in einer Verdünnung von 1:40, einmal mit einer 1:125 und beim zweiten Mal mit einer 1:250 Viruslysatverdünnung getestet. Die Ergebnisse sind in Fig. 1 aufgelistet. Zur weiteren Validierung des Testprinzips wurde eine größere Zahl von Seren (98 anti-HIV-1-positive und 76 anti-HIV-negative) auf inhibierende Antikörper mit rekombinanter RT (Verdünnung ca. 1:27 000) getestet. Das Gesamtergebnis mit der rekombinanten RT ist in Fig. 2 dargestellt.
c) Versuch zur prognostischen Aussagekraft RT-inhibierender Antikörper.
   Von einem Teil der 132 anti-HIV-1-positiven Seren war uns der klinische Zustand der Patienten bekannt (asymptomatisch LAS/ARC, AIDS) und bei einigen das Testergebnis im p24-Antigen-ETA (Fa. Abbott Laboratories, North Chicago, USA) im Serum oder Plasma. Bei einer dritten Gruppe kann das Reaktionsmuster im Westernblot (WB) für eine Einteilung in Zustand kurz nach Serokonversion (Frühinfektion), Patienten mit unauffälligem Antikörperprofil, beginnende Abschwächung von anti-p24 und Spätstadium benutzt werden. Um die prognostische Aussagekraft von RT-inhibierenden Serumantikörpern zu untersuchen, wurden jeweils der Anteil der Seren mit RT-Inhibition größer 50%, sowie die mittlere RT-Inhibition im Vergleich zwischen allen Gruppen berechnet (siehe Tabelle 2).

**Tabelle 2**

| Prognostische Aussagekraft RT-inhibierender Antikörper, im Vergleich mit anerkannten Prognoseparametern (p24-Antigenämie, klinische Einstufung, WB-Antikörperprofil). | | | | |
|---|---|---|---|---|
| | Anzahl getestet | Patienten mit > 50% RT-Inh. | entspricht % mit > 50% RT-Inh. | mittlere RT-Inh. in % |
| p24-Antigen positiv | 15 | 10 | 67 | 60,9 |
| p24-Antigen negativ | 38 | 34 | 89 | 77,3 |
| asymptomatisch | 29 | 25 | 86 | 79,1 |
| LAS/ARC | 10 | 9 | 90 | 78,3 |
| AIDS | 13 | 7 | 54 | 44,4 |
| Frühinfektion | 5 | 2 | 40 | 44,0 |
| WB unauffällig | 97 | 79 | 81 | 72,6 |
| Schwaches anti-p24 | 9 | 6 | 67 | 65,5 |
| WB-Spätstadium | 16 | 7 | 44 | 45,2 |

### Beispiel 5

### Nachweis von DNA-abhängiger DNA-Polymeraseaktivität

Klenow-Polymerase wird in einem Random Primed Labelling-Ansatz mit einem durch Hitze denaturierten DNA-Fragment und einem Hexamer-Gemisch, das aus Hexameren aller möglichen Sequenzen besteht, die jeweils als Primer dienen, inkubiert. Die Polymerase baut neben unmarkierten Nukleotiden DIGdUTP und BiodUTP ein. Die durch Polymeraseaktivität erzeugte doppelmarkierte DNA kann wiederum in Streptavidin-beschichteten ELISA-Platten quantitativ bestimmt werden.
a) Reaktionsansatz:
2 »g eines 356 bp langen DNA-Fragments werden in 15 »l H₂O 10 Minuten bei 95 °C denaturiert und anschließend sofort auf Eis gestellt. Zu diesen 15 »l werden pipettiert:
2 »l Hexanucleotid mixture in 10x reaction buffer
(aus Random Primed DNA Labelling Kit Best. Nr. 1004760, Boehringer Mannheim))
2 »l 10x Nucleotide-Mix
1 »l Klenow enzyme
(aus Random Primed DNA Labelling Kit Best. Nr. 1004760, Boehringer Mannheim)

| | | |
|---|---|---|
| 10x Nucleotide-Mix: | dATP | 2 mM |
| | dCTP | 2 mM |
| | dGTP | 2 mM |
| | dTTP | 1,75 mM |
| | DIGdUTP | 0,25 mM |
| | BiodUTP | 125 nM |

Der Reaktionsansatz wird 3 Stunden bei 37 °C inkubiert, anschließend wird er 1 : 10 mit TE-Puffer (10 mM Tris, 1 mM EDTA) verdünnt.
In einem Kontroll-Reaktionsansatz wird identisch verfahren, mit der Ausnahme, daß kein BiodUTP im Ansatz vorhanden ist.
b) Anlagerung der Reaktionsprodukte an Streptavidin in Mikrotiterplatten und Nachweis derselben
Jeweils 2 »l und 6 »l des verdünnten Reaktionsansatzes, die also 20 ng bzw. 60 ng des in die Markierungsreaktion eingesetzten DNA-Fragmentes enthalten, werden in 200 »l TE-Puffer in Streptavidinbeschichtete Mikrotiterplatten überführt und dort eine Stunde bei 37 °C inkubiert.
Anschließend werden die Platten dreimal mit 300 »l PBS-Puffer mit 0,5 % (v/v) Tween 20 gewaschen.
Danach werden 200 »l Anti-Digoxigenin-POD (150 mU/ml, Best. Nr. 1207733, Boehringer Mannheim) zugegeben. Nach einer Stunde Inkubation bei 37 °C wird wiederum dreimal mit je 300 »l PBS-Puffer mit 0,5 % (v/v) Tween 20 gewaschen. Anschließend werden 200 »l ABTS-Substratlösung wie in den anderen Beispielen zugegeben und nach 30 Minuten Inkubation die Farbentwicklung bei 405 nm gemessen.

| Ergebnis: | | | |
|---|---|---|---|
| verdünnter Reaktionsansatz ohne BiodUTP | | verdünnter Reaktionsansatz mit BiodUTP | |
| 2 »l | 6 »l | 2 »l | 6 »l |
| - 0,013 | 0,073 | 0,285 | 1,094 |

| Sequenzprotokoll | |
|---|---|
| SEQ ID NO: 1 | |
| Sequenzlänge: | 18 Basen |
| Art der Sequenz: | Nucleotid |
| Strangform: | Einzelstrang |
| Topologie: | linear |
| | GTCCCTGTTC GGGCGCCA |

## Patentansprüche

1. Verfahren zur Bestimmung von Polymerase-Aktivität welches folgende Schritte umfaßt:
- Inkubation einer Probe, welche die Polymerase enthält, mit einer Templat-Nukleinsäure und einem nachweisbaren Mononukleosidtriphosphat,
- Abtrennung der gebildeten Nukleinsäuren von dem nachweisbaren Mononukleosidtriphosphat, und
- Nachweis der in den gebildeten Nukleinsäuren enthaltenen nachweisbaren Nukleotide,
dadurch gekennzeichnet, daß
- die Inkubationsmischung zusätzlich ein immobilisierbares Mononukleosidtriphosphat enthält, und
- die Inkubationsmischung mit einer festen Phase in Kontakt steht oder gebracht wird, die sowohl das immobilisierbare Mononukleosidtriphosphat als auch die Nukleinsäuren, in welche das immobilisierbare Mononukleotid eingebaut ist, selektiv und im wesentlichen vollständig bindet.

2. Verfahren gemäß Anspruch 1, dadurch gekennzeichnet, daß die Templat-Nukleinsäure eine DNS ist, einen für die Polymerase spezifischen Promotor enthält und die Mononukleosidtriphosphate Ribonukleosidtriphosphate sind.

3. Verfahren gemäß Anspruch 1, dadurch gekennzeichnet, daß die Templatnukleinsäure eine DNS ist, der Inkubationsmischung zusätzlich ein Oligonukleotid zugegeben wird, das zu einem Teil der Templatnukleinsäure im wesentlichen komplementär ist, und die Mononukleosidtriphosphate Desoxyribonukleosidtriphosphate sind.

4. Verfahren gemäß Anspruch 1, dadurch gekennzeichnet, daß die Templat-Nukleinsäure eine RNS und die Mononukleosidtriphosphate Desoxyribonukleosidtriphosphate sind.

5. Verfahren gemäß Anspruch 1, dadurch gekennzeichnet, daß die Templat-Nukleinsäure eine RNS ist und eine Primer-Struktur enthält und die Mononukleosidtriphosphate Ribonukleosidtriphosphate sind.

6. Verfahren gemäß Anspruch 1, dadurch gekennzeichnet, daß der Inkubationsmischung ein Polymerase-Inhibitor zugesetzt wird.

7. Verfahren gemäß Anspruch 4, dadurch gekennzeichnet, daß es sich bei der Polymerase um eine reverse Transkriptase handelt.

8. Verfahren gemäß Anspruch 7, dadurch gekennzeichnet, daß die reverse Transkriptase aus HIV-Partikeln stammt.

9. Reagenzkit zum Nachweis von Polymeraseaktivität, enthaltend in getrennten Behältern:
- Templatnukleinsäure, mindestens ein nachweisbares und mindestens ein immobilisierbares Mononukleosidtriphosphat
- einen Behälter, an welchen das immobilisierbare Mononukleotid selektiv und im wesentlichen vollständig gebunden werden kann; und
- Waschlösungen.

10. Reagenzkit gemäß Anspruch 9, dadurch gekennzeichnet, daß es in einem weiteren Behälter Reagenzien zum Nachweis des nachweisbaren Mononukleotids enthält.

11. Reagenz für den Nachweis von Polymeraseaktivität enthaltend eine Templat-Nukleinsäure und ein nachweisbares Mononukleosidtriphosphat, dadurch gekennzeichnet, daß es zusätzlich ein immobilisierbares Mononukleotidtriphosphat enthält.

12. Verfahren zum Nachweis von HIV-RT dadurch gekennzeichnet, daß
- ein Lysat einer Probe, bei der die Anwesenheit von HIV vermutet wird
- dieses Lysat mit einer Templat-Ribonukleinsäure, mindestens einem nachweisbaren und einem immobilisierbaren Monodesoxyribonukleosidtriphosphat, einem Primer und Hilfsstoffen versetzt wird
- nach einer Inkubationszeit diese Mischung in einen Behälter überführt wird, der das immobilisierbare Mononukleotid und die dieses enthaltenden gebildeten Nukleinsäuren selektiv und im wesentlichen vollständig bindet,
- überschüssiges nachweisbares Mononukleosidtriphosphat abgetrennt wird und
- das gebundene nachweisbare Mononukleotid nachgewiesen wird.

13. Verfahren zur Feststellung der inhibierenden Wirkung einer Substanz auf Polymeraseaktivität durch
- Inkubation der Substanz mit einer Polymerase in Gegenwart einer Templatnukleinsäure und Mononukleosidtriphosphaten in Lösung und
- Nachweis der Menge der gebildeten Nukleinsäuren dadurch gekennzeichnet, dadurch gekennzeichnet daß
- in der Inkubationslösung ein nachweisbares und ein immobilisierbares Mononukleotidtriphosphat enthalten ist
- die Nukleinsäuren von überschüssigem, nicht eingebautem, nachweisbarem Mononukleosidtriphosphate abgetrennt werden und mit einer festen Phase, die sowohl das immobilisierbare Mononukleosidtriphosphat, als auch die Nukleinsäuren, in welche das immobilisierbare Mononukleotid eingebaut ist, selektiv und im wesentlichen vollständig bindet, in Kontakt gebracht werden.

14. Verfahren zum Nachweis eines Antikörpers gegen eine Polymerase durch
- Inkubation der Probe, in welcher der Antikörper nachgewiesen werden soll, mit einer Templatnukleinsäure, einem nachweisbaren Mononukleosidtriphosphat und der Polymerase,
- Abtrennung der gebildeten Nukleinsäuren von dem nachweisbaren Mononukleosidtriphosphat und
- Nachweis der in den gebildeten Nukleinsäuren enthaltenen nachweisbaren Nukleotide,
dadurch gekennzeichnet, daß
- die Inkubationsmischung zusätzlich ein immobilisierbares Mononukleosidtriphosphat enthält, und
- die Inkubationsmischung mit einer festen Phase in Kontakt steht oder gebracht wird, die sowohl das immobilisierbare Mononukleosidtriphosphat als auch die Nukleinsäuren, in welche das immobilisierbare Mononukleotid eingebaut ist, selektiv und im wesentlichen vollständig bindet.

15. Verfahren zum Nachweis einer Promotorsequenz, welches folgende Schritte umfaßt:
- Inkubation einer Probe, in welcher die Promotorsequenz nachgewiesen werden soll, mit einem nachweisbaren Mononukleosidtriphosphat und einer Polymerase, welche die Promotorsequenz als Initiator der Polymerasereaktion benötigt,
- Abtrennung der gebildeten Nukleinsäuren von dem nachweisbaren Mononukleosidtriphosphat und
- Nachweis der in den gebildeten Nukleinsäuren enthaltenen nachweisbaren Nukleotide, wobei
- die Inkubationsmischung zusätzlich ein immobilisierbares Mononukleosidtriphosphat enthält, und
- die Inkubationsmischung mit einer festen Phase in Kontakt steht oder gebracht wird, die sowohl das immobilisierbare Mononukleosidtriphosphat als auch die Nukleinsäuren, in welche das immobilisierbare Mononukleotid eingebaut ist, selektiv und im wesentlichen vollständig bindet.

16. Reagenzkit zum Nachweis eines Antikörpers gegen eine Polymerase, enthaltend in getrennten Behältern:
- Templatnukleinsäure sowie mindestens ein nachweisbares und mindestens ein immobilisierbares Mononukleosidtriphosphat;
- die Polymerase, gegen die der nachzuweisende Antikörper gerichtet ist.

## Claims

1. Process for the determination of polymerase activity which includes the following steps:
- incubation of a sample which contains the polymerase with a template nucleic acid and a detectable mononucleoside triphosphate,
- separation of the formed nucleic acids from the detectable mononucleoside triphosphate, and
- detection of the detectable nucleotides contained in the formed nucleic acids,
characterised in that
- the incubation mixture additionally contains an immobilisable mononucleoside triphosphate and
- the incubation mixture stands in or is brought into contact with a solid phase which selectively and substantially completely binds not only the immobilisable mononucleoside triphosphate but also the nucleic acids in which the immobilisable mononucleotide is incorporated.

2. Process according to claim 1, characterised in that the template nucleic acid is a DNA, contains a promotor specific for the polymerase and the mononucleoside triphosphates are ribonucleoside triphosphates.

3. Process according to claim 1, characterised in that the template nucleic acid is a DNA, to the incubation mixture is additionally added an oligonucleotide which is substantially complementary to a part of the template nucleic acid and the mononucleoside triphosphates are desoxyribonucleoside triphosphates.

4. Process according to claim 1, characterised in that the template nucleic acid is an RNA and the mononucleoside triphosphates are desoxyribonucleoside triphosphates.

5. Process according to claim 1, characterised in that the template nucleic acid is an RNA and contains a primer structure and the mononucleoside triphosphates are ribonucleoside triphosphates.

6. Process according to claim 1, characterised in that a polymerase inhibitor is added to the incubation mixture.

7. Process according to claim 4, characterised in that the polymerase is a reverse transcriptase.

8. Process according to claim 7, characterised in that the reverse transcriptase originates from HIV particles.

9. Reagent kit for the detection of polymerase activity containing, in separate containers:
- template nucleic acid, at least one detectable and at least one immobilisable mononucleoside triphosphate
- a container to which the immobilisable mononucleotide can be bound selectively and substantially complete; and
- wash solutions.

10. Reagent kit according to claim 9, characterised in that, in a further container, it contains reagents for the detection of the detectable mononucleotide.

11. Reagent for the detection of polymerase activity containing a template nucleic acid and a detectable mononucleoside triphosphate, characterised in that it additionally contains an immobilisable mononucleotide triphosphate.

12. Process for the detection of HIV-RT, characterised in that
- a lysate is prepared of a sample in the case of which the presence of HIV is suspected
- this lysate is mixed with a template ribonucleic acid, at least one detectable and an immobilisable monodesoxyribonucleoside triphosphate, a primer and adjuvant materials
- after an incubation time, this mixture is transferred into a container which selectively and substantially completely binds the immobilisable mononucleotide and the formed nucleic acids containing this
- excess detectable mononucleoside triphosphate is separated off and
- the bound detectable mononucleotide is detected.

13. Process for the ascertainment of the inhibiting action of a substance on polymerase activity by
- incubation of the substance with a polymerase in the presence of a template nucleic acid and mononucleoside triphosphates in solution and
- detection of the amount of the formed nucleic acids,
characterised in that
- in the incubation solution is contained a detectable and an immobilisable mononucleotide triphosphate
- the nucleic acids are separated off from excess, non-incorporated detectable mononucleoside triphosphate and brought into contact with a solid phase which selectively and substantially completely binds not only the immobilisable mononucleoside triphosphate but also the nucleic acids in which the immobilisable mononucleotide is incorporated.

14. Process for the detection of an antibody against a polymerase by
- incubation of the sample in which the antibody is to be detected with a template nucleic acid, a detectable mononucleoside triphosphate and the polymerase
- separation of the formed nucleic acids from the detectable mononucleoside triphosphate and
- detection of the detectable nucleotide contained in the formed nucleic acids,
characterised in that
- the incubation mixture additionally contains an immobilisable mononucleoside triphosphate and
- the incubation mixture stands in or is brought into contact with a solid phase which selectively and substantially completely binds not only the immobilisable mononucleoside triphosphate but also the nucleic acids in which the immobilisable mononucleotide is incorporated.

15. Process for the detection of a promotor sequence which includes the following steps:
- incubation of a sample in which the promotor sequence is to be detected with a detectable mononucleoside triphosphate and a polymerase which the promotor sequence requires as initiator of the polymerase reaction
- separation of the formed nucleic acids from the detectable mononucleoside triphosphate and
- detection of the detectable nucleotides contained in the formed nucleic acids, whereby
- the incubation mixture additionally contains an immobilisable mononucleoside triphosphate and
- the incubation mixture stands in or is brought into contact with a solid phase which selectively and substantially completely binds not only the immobilisable mononucleoside triphosphate but also the nucleic acids in which the immobilisable mononucleotide is incorporated.

16. Reagent kit for the detection of an antibody against a polymerase, containing in separate containers:
- template nucleic acid, as well as at least one detectable and at least one immobilisable mononucleoside triphosphate;
- the polymerase against which the antibody to be detected is directed.

## Revendications

1. Procédé pour la détermination de l'activité de polymérase, comprenant les étapes consistant à :
- incuber un échantillon qui contient la polymérase, avec une sonde nucléique et un mononucléosidetriphosphate détectable,
- séparer l'acide nucléique formé du mononucléosidetriphosphate détectable, et
- détecter les nucléotides détectables contenus dans les acides nucléiques formés,
caractérisé en ce que
- le mélange d'incubation contient en outre un mononucléosidetriphosphate fixé, et
- le mélange d'incubation est en contact ou est mis en contact avec une phase solide, à laquelle se lient, sélectivement et essentiellement complètement, le mononucléoside-triphosphate fixé ainsi que les acides nucléiques dans lesquels le mononucléotide fixé est incorporé.

2. Procédé selon la revendication 1, caractérisé en ce que la sonde nucléique est une sonde ADN, contient un promoteur spécifique à la polymérase et les mononucléosidetriphosphates sont des ribonucléotidetriphosphates.

3. Procédé selon la revendication 1, caractérisé en ce que la sonde nucléique est une sonde ADN, qu'on ajoute en plus au mélange d'incubation un oligonucléotide qui est essentiellement complémentaire d'une partie de la sonde nucléique, et les mononucléosidetriphosphates sont des désoxyribonucléotidetriphosphates.

4. Procédé selon la revendication 1, caractérisé en ce que la sonde nucléique est une sonde ARN et les mononucléosidetriphosphates sont des désoxyribonucléotidetriphosphates.

5. Procédé selon la revendication 1, caractérisé en ce que la sonde nucléique est une sonde ARN et contient une structure d'amorce, et les mononucléosidetriphosphates sont des ribonucléosidetriphosphates.

6. Procédé selon la revendication 1, caractérisé en ce qu'on ajoute au mélange d'incubation un inhibiteur de polymérase.

7. Procédé selon la revendication 4, caractérisé en ce que la polymérase est une transpcriptase réverse.

8. Procédé selon la revendication 7, caractérisé en ce que la transpcriptase réverse est issue de particules de HIV.

9. Kit de réactifs pour la détection de l'activité de polymérase, contenant, dans des récipients séparés :
- des sondes nucléiques, au moins un mononucléosidetriphosphate détectable et au moins un mononucléosidetriphosphate fixé,
- un récipient auquel le mononucléosidetriphosphate fixé peut être lié sélectivement et de façon essentiellement complète ;
- des solutions de lavage.

10. Kit de réactifs selon la revendication 9, caractérisé en ce qu'il contient, dans un récipient supplémentaire, des réactifs pour la détection de mononucléotides détectables.

11. Réactif pour la détection d'activité de polymérase, contenant une sonde nucléique et un mononucléosidetriphosphate détectable, caractérisé en ce qu'il contient en outre un mononucléosidetriphosphate fixé.

12. Procédé pour la détection de HIV-RT, caractérisé en ce que
- au lysat d'un échantillon, dans lequel on suppose la présence de HIV,
- on ajoute une sonde ribonucléique, au moins un monodésoxyribonucléosidetriphosphate détectable et un monodésoxyribonucléosidetriphosphate fixé, une amorce et des adjuvants,
- après une période d'incubation, on transfert ce mélange dans un récipient, auquel se fixent sélectivement et de façon essentiellement complète le mononucléotide fixé et les acides nucléiques formés qui le contiennent,
- on sépare les mononucléosidetriphosphates détectables en excès, et
- on détecte le mononucléotide détectable fixé.

13. Procédé pour la détermination de l'activité inhibitrice d'une substance vis-à-vis de l'activité de polymérase, comprenant les étapes consistant à :
- incuber la substance avec une polymérase en présence d'une sonde nucléique et de mononucléosidetriphosphates en solution, et
- détecter la quantité d'acides nucléiques formés,
caractérisé en ce que
- la solution d'incubation contient un mononucléosidetriphosphate détectable et un mononucléosidetriphosphate fixé,
- on sépare les acides nucléiques des mononucléosidetriphosphates détectables en excès, non incorporés, et on les met en contact avec une phase solide, à laquelle se lient, de manière sélective et essentiellement complète, les mononucléosidetriphosphates fixés ainsi que les acides nucléiques dans lesquels est incorporé le mononucléotide fixé.

14. Procédé de détection d'un anticorps dirigé contre une polymérase, comprenant les étapes consistant à :
- incuber un échantillon, dans lequel l'anticorps doit être détecté, avec une sonde nucléique, un mononucléosidetriphosphate détectable et la polymérase,
- séparer les acides nucléiques formés du mononucléosidetriphosphate détectable, et
- détecter les nucléotides détectables contenus dans les acides nucléiques formés,
caractérisé en ce que
- le mélange d'incubation contient en outre un mononucléosidetriphosphate détectable, et
- le mélange d'incubation est en contact ou est mis en contact avec une phase solide, à laquelle se lent sélectivement et essentiellement complètement le mononucléosidetriphosphate fixé ainsi que les acides nucléiques dans lesquels le mononucléotide fixé est incorporé.

15. Procédé de détection d'une séquence de promoteur, comprenant les étapes consistant à :
- incuber un échantillon, dans lequel le promoteur doit être détecté, avec un mononucléosidetriphosphate détectable et une polymérase qui nécessite le promoteur pour initier la réaction de polymérase,
- séparer du mononucléosidetriphosphate détectable les acides nucléiques formés, et
- détecter les nucléotides détectables contenus dans les acides nucléiques formés, procédé dans lequel
- le mélange d'incubation contient en outre un mononucléosidetriphosphate fixé, et
- le mélange d'incubation est en contact ou est mis en contact avec une phase solide, à laquelle se lient, sélectivement et essentiellement complètement, le mononucléosidetriphosphate fixé ainsi que les acides nucléiques dans lesquels le mononucléotide fixé est incorporé.

16. Kit de réactifs pour la détection d'un anticorps dirigé contre une polymérase, contenant, dans des récipients séparés :
- des sondes nucléiques ainsi qu'au moins un mononucléosidetriphosphate détectable et un mononucléosidetriphosphate fixé ;
- la polymérase, contre laquelle est dirigé l'anticorps à détecter.
